# EUROPEAN PATENT SPECIFICATION

(11) **EP 1 343 550 B1**
(45) Date of publication and mention of the grant of the patent: **22.11.2017**
(21) Application number: 01272108.0
(22) Date of filing: 21.12.2001
(51) Int. Cl.: A61M 15/00, A61K 9/00, B65D 83/14

(54) **METERED DOSE INHALER FOR SALMETEROL XINAFOATE**
DOSIERINHALATOR FÜR SALMETEROL XINAFOATE
AEROSOL-DOSEUR POUR XINAFOATE DE SALMETEROL

(30) Priority: 22.12.2000 GB 0031502; 29.11.2001 GB 0128612
(43) Date of publication of application: 17.09.2003
(73) Proprietor: Glaxo Group Limited, Brentford, Middlesex TW8 9GS (GB)
(72) Inventor: GODFREY, Anne, Pauline, Ware, Hertfordshire SG12 0DP (GB); WARBY, Richard, Kings Lynn, Norfolk PE30 2JJ (GB)
(74) Representative: Thompson, Clive Beresford
(86) International application number: PCT/GB2001/005749
(87) International publication number: WO 2002/051483

(56) References cited:
- EP-A- 0 990 437
- WO-A-00/37336
- WO-A-00/56632
- WO-A-01/76601
- WO-A-95/02651
- WO-A-96/32150
- WO-A-99/47195
- US-A- 6 089 256

## Description

The invention provides a container for a metered dose inhaler (MDI) for use in dispensing a quantity of a pharmaceutical formulation, especially salmeterol xinafoate formulation, which may be used in the treatment of respiratory disorders.

4-hydroxy-α¹-[[[6-(4-phenylbutoxy)hexyl]amino]methyl]-1,3-benzenedimethanol was described as one of a wide range of bronchodilators in GB-A-2140800. This compound is also known by the generic name of salmeterol, the 1-hydroxy-2-naphthoate (xinafoate) salt of which has become widely known as a highly effective treatment of inflammatory diseases, such as asthma and chronic obstructive pulmonary disease (COPD).

Containers for aerosol formulations commonly comprise a vial body (canister) coupled to a valve. The valve comprises a valve stem through which the formulations are dispensed. Generally the valve includes a rubber valve seal intended to allow reciprocal movement of the valve stem which prevents leakage of propellant from the container. Metered dose inhalers comprise a valve which is designed to deliver a metered amount of an aerosol formulation, to the recipient, per actuation. Such a metering valve generally comprises a metering chamber which is of a set volume which aims to administer per actuation an accurate, predetermined dose.

Metering valves incorporate gaskets (also known as seals) to prevent leakage of propellant through the valve. The gasket may comprise suitable elastomeric material such as for example low density polyethylene, chlorobutyl, black and white butadiene-acrylonitrile rubbers, butyl rubber and neoprene.

Valves for use in MDIs are available from manufacturers well known in the aerosol industry, for example, from Valois, France (eg. DF10, DF30, DF60), Bespak plc, United Kingdom (eg. BK300, BK356, BK357) and 3M-Neotechnic Limited, United Kingdom (eg. Spraymiser™). The metering valves are used in association with commercially available canisters, for example metal canisters, such as aluminium canisters, suitable for delivering pharmaceutical aerosol formulations.

MDIs incorporating valve gaskets as described above perform adequately with chlorofluorocarbon propellants such as propellant 11 (CCl₃F), propellant 114 (CF₂ClCF₂Cl) and propellant 12 (CCl₂F₂) or mixtures thereof. However these propellants are now believed to provoke the degradation of stratospheric ozone and there is thus a need to provide aerosol formulations for medicaments which employ so called "ozone-friendly" propellants.

A class of propellants which are believed to have minimal ozone-depleting effects in comparison to conventional chlorofluorocarbon propellants comprises hydrofluoroalkanes (HFA's) especially 1,1,1,2-tetrafluoroethane (HFA134a), 1,1,1,2,3,3,3-heptafluoro-n-propane (HFA 227) and mixtures thereof. However there have been problems associated with stabilising the pharmaceutical aerosol formulations prepared using the new class of propellants.

Pharmaceutical aerosol formulations may comprise a solution or a suspension. Some solution formulations suffer the disadvantage that the drug substance contained therein is more susceptible to degradation. Furthermore there are issues with control of size of the droplets which influences the therapeutic profile. For this reason, suspensions are generally preferred.

Suspension aerosol formulations generally comprise a particulate medicament, one or more liquid propellants, optionally with a co-propellant, and optionally an adjuvant such as a solvent or a surfactant. The aerosol formulation is under pressure in the canister.

The efficiency of an aerosol device, such as an MDI, is a function of the dose deposited at the appropriate site in the lungs. Deposition is affected by several factors, of which one of the most important is the aerodynamic particle size. Solid particles and/or droplets in an aerosol formulation can be characterised by their mass median aerodynamic diameter (MMAD, the diameter around which the mass aerodynamic diameters are distributed equally).

In suspension formulations, particle size in principle is controlled during manufacture by the size to which the solid medicament is reduced, usually by micronisation. However, if the suspended drug has the slightest solubility in propellant, a process known as Ostwald Ripening can lead to particle size growth. Also, particles may have the tendency to aggregate, or adhere to parts of the MDI eg. canister or valve. Furthermore the drug may have the tendency to be absorbed into the rubber components of the valve, especially when stored for a prolonged period. In particular fine particles may be preferentially absorbed. The effect of Ostwald Ripening and especially of drug deposition may be particularly severe for potent drugs (including salmeterol xinafoate) which need to be formulated in low doses.

The problems mentioned above have been addressed by the addition of one or more of adjuvants such as alcohols, alkanes, dimethyl ether, surfactants (including fluorinated and non-fluorinated surfactants, carboxylic acids, polyethoxylates etc) and even conventional chlorofluorocarbon propellants in small amounts intended to minimise potential ozone damage as disclosed in, for example, EP0372777, WO91/04011, WO91/11173, WO91/11495 and WO91/14422.

Excipient free formulations of salmeterol xinafoate are described in WO93/11743.

Furthermore, WO96/32345, WO96/32151, WO96/32150 and WO96/32099 disclose aerosol canisters coated with one or more fluorocarbon polymers optionally in combination with one or more non-fluorocarbon polymers.

It is essential that the prescribed dose of aerosol medication delivered from MDIs to the patient consistently meet the specifications claimed by the manufacturer and comply with the requirements of the FDA and other regulatory authorities. That is, every dose delivered from the can must be the same within close tolerances. Therefore it is important that the formulation be substantially homogeneous throughout the administered dose throughout the life of the product. It is also important that the concentration of the suspension does not change significantly when stored for a prolonged period.

To obtain regulatory approval, pharmaceutical aerosol formulation products must meet strict specifications. One parameter for which a specification is usually set is the fine particle mass (FPM). This is a means of evaluating the amount of drug substance which has the potential to reach the inner lungs, i.e. the small bronchioles and alveoli, based on the amount of drug particles with a diameter within a certain range, usually less than 5 microns.

The FPM of an actuation from an MDI can be calculated based on the sum of the amount of drug substance deposited on stages 3, 4 and 5 of an Andersen Cascade Impaction stack as determined by standard HPLC analysis.

It is important that the FPM of the pharmaceutical aerosol formulation for all the doses dispensed from the MDI are within the specification set, even after the MDI has been stored for a prolonged period.

Whilst not wishing to be bound by any theories it is hypothesised by the inventors that the concentration of drug in the suspension and thus the dose dispensed may, in many cases (especially in particulate salmeterol xinafoate and HFA formulations), decrease over time with the adsorption of drug into the rubber components of the valve. This may be observed as a decrease in the Total Drug Content (TDC) of the can. This process may be accelerated by the ingression of water into the formulation.

This hypothesis has been supported by studies employing salmeterol xinafoate HFA 134a aerosol formulations in conventional MDI's stored at 40°C and 75% relative humidity and 40°C and 20% relative humidity as shown in Table 1.

Furthermore evidence indicates that the FPM and mean dose of some particulate aerosol formulations, for example, salmeterol xinafoate HFA 134a formulations decreases over time with the ingression of water into the formulation and/or deposition and/or absorption resulting in impaired performance of the MDI.

The effect on FPM of salmeterol xinafoate HFA 134a aerosol formulations in conventional MDIs stored at 40°C and 75% relative humidity is shown in Table 2 and Table 4. Table 3 and Table 5 show a noticeable decrease over time in the mean dose delivered from a conventional MDI when stored at 40°C and 75% relative humidity.

Deposition of drug particles on other valve components, particularly the metering chamber may also contribute to the formulation stability problems observed such as inconsistencies in the doses dispensed, which becomes particularly acute over increasing numbers of actuations.

The problem with deposition is particularly exacerbated when excipient-free aerosol formulations are used based on the propellants 1,1,1,2-tetrafluoroethane (HFA 134a) and 1,1,1,2,3,3,3-heptafluoro-n-propane (HFA 227) and is thought to increase with length of storage of the aerosol, particularly when stored at high temperature and/or high humidity.

Surprisingly the inventors have found that the suspension concentration, dose and FPM of formulations of particulate medicament suspended in a HFA propellant e.g. salmeterol xinafoate in suspension in a HFA propellant, obtained from an MDI may be stabilised by reducing the deposition on the valve component(s) and reducing the drug absorption into rubber components and/or effectively controlling the ingression of water into the formulation during storage and use by employing particular valve materials.

Thus the invention provides a container comprising a canister sealed with a metering valve having a metering chamber, which contains a pharmaceutical aerosol formulation consisting essentially of
(A) particulate salmeterol xinafoate optionally in combination with another drug useful in inhalation therapy, suspended in
(B) a liquefied propellant gas comprising 1,1,1,2,3,3,3-heptafluoro-n-propane, 1,1,1,2-tetrafluoroethane or a mixture thereof;
wherein the formulation contains less than 0.1%surfactant based on weight of medicament and contains less than 0.1% components having polarity higher than the liquefied propellant gas based on weight of medicament;
said valve characterised in that it contains one or more sealing gaskets substantially constructed from of a polymer of ethylene propylene diene monomer (EPDM) (wherein substantially constructed means a gasket composed of greater than 90% EPDM) and the metering chamber surface presents a substantially fluorinated surface to the formulation.

Preferably the formulation will consist of particulate salmeterol xinafoate optionally in combination with another drug useful in inhalation therapy, suspended in 1,1,1,2,3,3,3-heptafluoro-n-propane, 1,1,1,2-tetrafluoroethane or a mixture thereof.

More preferably the liquefied propellant gas is 1,1,1,2-tetrafluoroethane.

We have found that the trends observed, especially for salmeterol xinafoate, wherein the dose delivered and the FPM is reduced after storage, especially at elevated temperatures and under high humidity conditions, can be ameloriated by use of one or more gaskets constructed substantially from a polymer of EPDM. However the absolute values of the dose delivered and the FPM are not significantly increased. Therefore to ensure the patient gets the correct dose each time the device is actuated the formulation must contain an excess of drug substance, sometimes called "overage", to compensate for the loss. Advantageously when said gaskets are used in conjunction with a metering chamber which presents a substantially fluorinated surface to the formulation, the absolute dose of medicament available to the patient is raised whilst simultaneously maintaining or improving the stabilisation in the dose delivered and FPM. This provides benefits to the patient who receives the full dose claimed to be available on the label of the medicament and is more likely to satisfy the rigorous standards of the FDA and other regulatory authorities. Furthermore there are economic advantages since wastage of product is reduced.

Furthermore water is repelled from the fluorinated surface of the metering chamber which may further reduce the water ingression into the formulation over time, thereby reducing the undesirable effects thereof.

A particular aspect of the invention is a container as described above wherein the valve is sealed to the canister by means of a can/neck sealing gasket (3) which is substantially constructed from a polymer of EPDM.

Especially preferred is a container as described above wherein the metering valve comprises a metering chamber (4) defined by walls and an upper (12) and a lower (9) sealing gasket through which pass a valve stem (7 and 8) characterised in that said two sealing gaskets are substantially constructed from a polymer of EPDM and the metering chamber surface presents a substantially fluorinated surface to a formulation containable therein.

Also especially preferred is a container as described above wherein the valve is sealed to the canister by means of a can sealing gasket (3) which is substantially constructed from EPDM polymer and wherein the lower (9) sealing gasket is substantially constructed from EPDM polymer.

Most preferably all the sealing gaskets in the said metering valve are substantially constructed from EPDM polymer.

In the foregoing, the expressions "polymer of EPDM" and "EPDM polymer" are used interchangeably.

Sealing gasket when used in this specification will be understood to mean a neck/canister gasket and/or lower sealing gasket and/or upper sealing gasket.
Figure 1 shows part of a cross-section view of an MDI, with the valve pointing downward. The gaskets are represented by: 3 the can/neck seal, 9 the lower metering chamber seal and 12 the upper metering chamber seal. The metering chamber is identified as 4 and the stem is identified as 7 and 8.
Figure 2 shows an alternative cross-section of an MDI valve.

EPDM polymer when used as a gasket material in valves for use with aerosol formulations of particulate medicament in a HFA propellant appears to reduce deposition of drug particles on said gaskets in comparison to those gaskets prepared from traditional materials.

Furthermore EPDM polymer properties have been found to be superior to those materials traditionally used with respect to the absorption of drug into rubber.

In addition it seems that EDPM polymer may also have superior properties with respect to the control of water ingression into the pharmaceutical aerosol formulation containing hydrofluorocarbons. This is illustrated in Table 2 which shows that salmeterol xinafoate HFA 134a formulations in MDIs with gaskets of EPDM polymer have a stable FPM and dose delivered at the beginning of use even when stored at 40°C and relative humidity 75% for up to 6 months.

Table 3 and Table 5 give mean dose data and range of dose data for beginning of use which further supports the improved stability of formulations illustrated by salmeterol xinafoate HFA 134a formulations wherein the valve gaskets are composed of EPDM polymer.

In addition it seems that the life span of the gaskets of EPDM polymer is longer than that of traditional gaskets as the material is more stable to the hydrofluorocarbon containing formulations and more resistant to degradation and/or distortion. Therefore the advantages of the EPDM polymer are enjoyed throughout the life of the product without the function of the device being impaired.

EPDM polymer is available from a variety of suppliers including West and Parker Seals (USA).

A gasket substantially constructed from a polymer of EPDM when used in this specification will be understood to mean a gasket composed of greater than 90% of EPDM polymer, particularly greater than 95% of EPDM polymer, especially greater than 99% of EPDM polymer.

The invention also relates to a container as described above wherein the metering chamber presents a substantially fluorinated surface to the formulation. This advantageously reduces drug deposition on the metering chamber when used in conjunction with aerosol HFA formulations such as salmeterol xinafoate HFA formulations, compared with valves conventionally available.

The said metering chamber may be constructed from of any material with suitable characteristics such as any conventionally used plastics material such as nylon, polybutylene terephthalate PBT (polyester), acetal (polyoxymethylene) and tetrabutyrene terephthalate (TBT) etc, or metallic material which is compatible for use with the formulation, for example, stainless steel or aluminium. One example of a metal valve is the 3M-Neotechnic valve.

The metering chamber (especially when composed of a plastics material) is preferably surface treated so as to present a substantially fluorinated surface to the formulation.

Preferably surface treatment will comprise a process of plasma coating with highly fluorinated small molecules such as: C₁₋₁₀perfluoroalkanes including perfluorocycloalkanes; C₂₋₁₀perfluoroalkenes; fluoroalkanes including fluorocycloalkanes or fluoroalkenes wherein a high proportion of the hydrogens have been replaced by fluorines or mixtures thereof. Furthermore the fluorinated molecules or mixtures thereof may optionally be used in combination with one or more non-fluorocarbon compounds.

Especially preferred small molecules include C₁₋₁₀perfluoroalkanes.

The plasma coating may comprise a fluorinated polymer laid down on the surface of the valve component, preferably the metering chamber, by polymerisation or direct modification of the material surface by interchange of hydrogen ions in the material with fluorine ions. The coating process typically takes place in a vacuum at ambient temperature. The components to be coated are placed inside a chamber which is evacuated. The fluorine monomer or fluorine source is introduced into the chamber at a controlled rate. The plasma is ignited within the chamber and maintained for a given time at a chosen power setting. At the end of the treatment the plasma is extinguished, the chamber flushed and the products retrieved. In the polymerisation process, a thin layer of plasma polymer will be bonded to the surface of the valve component, preferably a metering chamber, or any other surface of the valve to be coated.

For plasma polymerization typically temperatures in the range of about 20°C to about 100°C may be employed.

The surface of the component especially the metering chamber may require activating in order to facilitate more effective coating of the surface by improving the adhesion of the coating to the surface.

Preferably the components to be plasma coated will be pre-treated to remove any surface contamination and/or to activate the surface. This may be achieved by, for example, treatment of the components with an etching gas such as oxygen or argon. In the process radicals react with the plastic or metal substrate e.g the component is exposed to a low pressure oxygen plasma environment which creates polar groups on the components surface which are more conducive to bonding with the plasma coating to be applied.

Alternatively the metering chamber (especially when composed of a plastics material, for example, those described above) may be surface treated with a siloxane such as dimethyl siloxane using a similar process as that described above for fluoroplasma coating.

Alternatively the metering chamber presents a substantially fluorinated surface to the formulation by virtue of being composed of a suitable substantially fluorinated material.

Suitable fluorinated materials include fluorinated polymer/copolymer or mixtures thereof or a mixture of the fluorinated polymer in combination with non-fluorinated polymers conventionally used in the manufacture of valves, such as acetal, polyester (PBT).

Examples of suitable fluorinated polymers include polytetrafluoroethylene (PTFE), ethylenetetrafluoroethylene (ETFE), polyvinyldienefluoride (PVDF), perfluoroalkoxyalkane (PFA), polyvinylfluoride (PVF), polychlorotrifluoroethylene (PCTFE), fluorinated ethylenepropylene (FEP) etc. Suitable copolymers include copolymers of tetrafluoroethylene (TFE) with PFA, TFE with hexafluoropropylene (HFP) (available as FEP 6107 and FEP 100 from DYNEON), VDF with HFP (commercially available as Viton A), TFE with perfluoro(propyl vinyl ether) (available as PFA 6515N from DYNEON), a blend of TFE, hexafluoropropylene and vinylidene fluoride (available commercially as THV 200G from DYNEON), etc.

It should be noted, however, that any conventionally available polymer, copolymer or mixture thereof which comprises a fluorinated polymer and which can be used to make the valve for use in an inhaler according to the invention may be suitable. Examples of mixtures of polymers and/or copolymers comprise, for example, up to 80% by weight fluorinated polymer, optionally up to 40% by weight fluorinated polymer, optionally up to 20% by weight fluorinated polymer or optionally up to 5% by weight of fluorinated polymer. Preferably, fluorinated polymers selected from PTFE, PVF and PCTFE are used as mixtures with non-fluorinated polymers. For example a suitable material is HOSTAFORM X329™ (Hoechst) which is a 5% PTFE/Acetal blend, HOSTAFORM C9021TF which is a 20% PTFE/Acetal blend, PTFE/PBT blends (for example, LNP WL4040), PTFE/PBT/silicone blends (for example, LNP WL4540).

The fluorinated polymers and mixtures thereof used in the invention can be moulded in any conventional manner, for example, by injection moulding, plastic moulding etc.

Alternatively metering chambers (especially when composed of a metallic material such as aluminium or stainless steel) can be coated by conventional techniques using fluorocarbon polymers which include fluorocarbon polymers which are made of multiples of one or more of the following monomeric units: tetrafluoroethylene (PTFE), fluorinated ethylene propylene (FEP), perfluoroalkoxyalkane (PFA), ethylene tetrafluoroethylene (ETFE), vinyldienefluoride (PVDF), and chlorinated ethylene tetrafluoroethylene. Fluorinated polymers, which have a relatively high ratio of fluorine to carbon, such as perfluorocarbon polymers, e.g. PTFE, PFA or FEP may be preferable, especially polymers selected from PTFE and FEP.

The metering chamber may be treated, so as to present a substantially fluorinated surface to the formulation, for example, by coating with a fluorinated polymer which is optionally blended with non-fluorinated polymers such as polyamides, polyimides, polyamideimides, polyethersulfones, polyphenylene sulfides, and amine-formaldehyde thermosetting resins. These added polymers often improve adhesion of the polymer coating to the substrate. Preferred polymer blends are PTFE/FEP/polyamideimide, PTFE/ polyethersulphone (PES) and FEP-benzoguanamine. The most preferred polymer coating is a blend of PTFE and PES. A coating of pure FEP is also of considerable interest.

A technique for applying such coatings to, for example, a metal, such as aluminium or stainless steel, is where the metal is precoated as coil stock and cured before being stamped or drawn into the can shape. This method is well suited to high volume production for two reasons. First, the art of coating coil stock is well developed and several manufacturers can custom coat metal coil stock to high standards of uniformity and in a wide range of thicknesses. Second, the precoated stock can be stamped or drawn at high speeds and precision by essentially the same methods used to draw or stamp uncoated stock.

Other techniques for coating techniques includes electrostatic dry powder coating or by spraying preformed MDI components with formulations of the coating fluorinated polymer/polymer blend and then curing. The preformed MDI components may also be dipped in the fluorocarbon polymer/polymer blend coating formulation and cured, thus becoming coated on the inside and out. The fluorocarbon polymer/polymer blend formulation may also be poured inside the MDI components then drained out leaving the insides with the polymer coat.

The appropriate curing temperature is dependent on the polymer blend chosen for the coating and the coating method employed.

However, for coil coating and spray coating temperatures in excess of the melting point of the polymer are typically required, for example, about 50°C above the melting point for up to about 20 minutes such as about 5 to 10 minutes e.g. about 8 minutes or as required. For the above named preferred and particularly preferred polymer blends curing temperatures in the range of about 300°C to about 400°C, e.g. about 350°C to 380°C are suitable.

Where the components are coated and then cured the substrate components may be prepared from strengthened materials to ensure they withstand the process.

Thus an aspect of the invention is a process for preparing a container, as described above, wherein the surface treatment of the metering chamber comprises a process for applying a coating of a fluorocarbon polymer optionally in combination with a non-fluorocarbon polymer.

Conversely alternative polymer coatings may be used on the components which may be dipped or bath immersed into a treatment tank containing a solution of polymeric compound. Usually the components are immersed in the solution at room temperature for at least one hour, for example, 12 hours, thus being treated both internally and externally.

The treated components are preferably washed with solvent and dried at an elevated temperature for example 50-100°C optionally under vacuum.

Examples of suitable coating materials include of fluoropolyethers having functionalised ends groups with a general formula R_{f}-O(C₃F₆O)ₘ(CFX)ₙ-CFX-Y-Zₚ as described in USP 4, 746, 550 (incorporated herein by reference) including perfluoropolyethers having functional groups capable of anchoring the coating to the substrate such as carboxyl, ester, amide, hydroxyl, isocyanate, epoxy, silane, for example, -CONR²R³ wherein R² and R³ may be independently selected from amongst other things hydrogen, or a silyl ether (e.g. SiRₜ(OR)₃₋ₜ or a fluoropolyether having hydroxylic functionality of the type -CF₂CH₂OH, -CF₂CFXCH₂OH (wherein X is Cl or F) or -CF(CF₃)CH₂OH as described in USP 6, 071, 564 (incorporated herein by reference); phosphoric diesters of formula [XCF₂CF₂O(CFXCF₂O)ₓCFXCH₂O]₂PO(OM) as described in USP 3, 492, 374 (incorporated herein by reference) or phosphoric monoester of formula [R_{f} -O-CFY-L-O]ₘP=O(O⁻Z⁺)₃₋ₘ as described in EP 0 687 533 (incorporated herein by reference) wherein L is a divalent organic group; m = 1; Y is -F or -CF₃; Z⁺ is selected from H⁺, M⁺ where M is an alkali metal; N(R)₄⁺ where the R groups independently represent H or C₁₋₆alkyl; R_{f} is a polyperfluoroalkyleneoxide chain.

The fluoropolyethers described above may be used in combination with monofunctional fluoropolyethers having -CH₂OH terminals directly linked to a perfluoroalkyl group -CF₂, -CF₂CFX (wherein X is Cl or F) or CF(CF₃) optionally through a bridging group (CH₂CH₂)_{q} wherein q represents an integer from 1 to 6.

Other suitable coating materials also include polymeric compounds that are silane derivatives of perfluoropolyoxyalkanes with a molecular weight in the range 1600-1750 and those of the general formula:

R¹-(CH₂)ᵥ-CF₂O-(C₂F₄O)ₓ-(CF₂O)_{y}CF₂-(CH₂)_{w}-R¹ (I)

wherein R¹ comprises:
-(OCH₂-CH₂)₂-OPO(OH)₂, wherein x, y and z are such that the molecular weight of the compound is 900-2100 and v and w independently represent 1 or 2.

The invention also relates to a container as described above wherein the valve stem presents a substantially fluorinated surface to the formulation.

Stems to be plasma coated may optionally be pretreated to remove surface contamination and/or activate the surface.

Alternatively stems may be coated by conventional techniques using fluorocarbon polymers optionally in combination with non-fluorocarbon polymer wherein the said coating requires curing after application as described above.

Additionally stems may be coated by processes using fluorocarbon polymers that require drying at temperatures between 50-100°C as described above for metering chambers.

Alternatively the stem presents a substantially fluorinated surface to the formulation by virtue of being composed of a suitable fluorinated material.

Analogous processes and materials described above for metering chambers are suitable for the preparation of valve stems according to the invention.

Preferably the substantially fluorinated surface will result from surface treatment of the stem. Most preferably the surface treatment will comprise a process of plasma coating with highly fluorinated small molecules such as: C₁₋₁₀perfluoroalkanes including fluorocycloalkanes; C₂₋₁₀perfluoroalkenes; fluoroalkanes including fluorocycloalkanes or fluoroalkenes wherein a high proportion of the hydrogens have been replaced by fluorines or mixtures thereof as described above.

Preferably the container according to the invention is a canister composed of aluminium.

Preferably the canister also presents a substantially fluorinated surface to the formulation.

Preferably the canister is surface treated so as to present a substantially fluorinated surface to the formulation contained therein.

More preferably the canister is surface treated by coating with a fluorocarbon polymer optionally in combination with a non-fluorocarbon polymer, for example, using materials mentioned above. Fluorocarbon polymers selected from FEP and PTFE are particularly preferred for the surface treatment of canisters. FEP is especially preferred. A polymer blend of PTFE and PES is also especially preferred.

The surface treatment of the canister may be performed by methods analogous to those described above for valve components.

Preferably salmeterol xinafoate is the only medicament. However medicaments which may be administered in aerosol formulations according to the invention in combination with salmeterol xinafoate include any drug useful in inhalation therapy e.g; anti-allergics, e.g. cromoglycate (e.g. as the sodium salt), ketotifen or nedocromil (e.g. as sodium salt); anti-inflammatory steroids, e.g. beclomethasone (e.g. as dipropionate), fluticasone (e.g. as propionate), flunisolide, budesonide, rofleponide, mometasone (e.g as furoate), ciclesonide, triamcinolone acetonide; anticholinergics, e.g. ipratropium (e.g. as bromide), tiotropium, atropine or oxitropium and salts thereof. It will be clear to a person skilled in the art that, where appropriate, the medicaments may be used in the form of salts, (e.g. as alkali metal or amine salts or as acid addition salts) or as esters (e.g. lower alkyl esters) or as solvates (e.g. hydrates) to optimise the activity and/or stability of the medicament and/or to minimise the solubility of the medicament in the propellant.

Specific medicaments of interest for use in combination with salmeterol xinafoate include fluticasone propionate or ipratropium bromide.

The container and valve described herein may also be suitable for containing medicaments besides salmeterol xinafoate which present similar formulation difficulties e.g. because of their susceptibility to water ingress, drug deposition, and other drug losses. Generally these difficulties are especially severe for potent medicaments which are administered at low doses. Examples include salmeterol and salts thereof, fluticasone propionate, formoterol and salts thereof. Other example medicaments include beclomethasone dipropionate, budesonide, sodium cromoglycate, albuterol and salts thereof and combinations thereof.

Medicament may be used in the form of racemate or in the form of a pure isomer e.g. R-salmeterol or S-salmeterol.

The particle size of the particulate (e.g. micronised) medicament should be such as to permit inhalation of substantially all of the medicament into the lungs upon administration of the aerosol formulation and will thus be less than 100 microns, desirably less than 20 microns, and preferably in the range 1-10 microns, e.g. 1-5 microns.

The concentration of medicament in the formulation will generally be 0.01-10% such as 0.01-2%, particularly 0.01-1%, especially 0.03-0.25% w/w. When salmeterol xinafoate is the only medicament its concentration in the formulation will generally be 0.03-0.15% w/w.

It is desirable that the formulations of the invention contain no components which may provoke the degradation of stratospheric ozone. In particular it is desirable that the formulations are substantially free of chlorofluorocarbons such as CCl₃F, CCl₂F₂ and CF₃CCl₃. It is desirable that the formulations of the invention are substantially free of any volatile adjuvant such as a saturated hydrocarbon, for example, propane, n-butane, isobutane, pentane and isopentane or a dialkyl ether, for example, dimethyl ether.

It is desirable that the formulations of the invention are substantially free of surfactant. "Substantially free" will generally be understood to mean containing less than 0.01% w/w especially less than 0.0001% based on weight of medicament.

It is desirable that the formulations of the invention are substantially free of any polar adjuvants e.g. C₂₋₆aliphatic alcohols and polyols such as ethanol, isopropanol propylene glycol, glycerol and mixtures thereof. "Substantially free" will generally be understood to mean containing less than 0.01% especially less than 0.0001% based on weight of formulation. Polarity may be determined, for example, by the method described in European Patent Application Publication No. 0327777.

Thus in one aspect the invention provides a container which contains a pharmaceutical aerosol formulation comprising a particulate medicament and a liquefied propellant gas of 1,1,1,2,3,3,3-heptafluoro-n-propane, 1,1,1,2-tetrafluoroethane or mixtures thereof. Preferably the pharmaceutical aerosol formulation will consist of or consist essentially of a particulate medicament and a liquefied propellant gas of 1,1,1,2,3,3,3-heptafluoro-n-propane, 1,1,1,2-tetrafluoroethane or mixtures thereof.

Most preferably the propellant gas is 1,1,1,2-tetrafluoroethane.

The term "metered dose inhaler" or MDI means a unit comprising a canister, a secured cap covering the canister and a formulation metering valve situated in the cap. MDI system includes a suitable channelling device. Suitable channelling devices comprise, for example, a valve actuator and a cylindrical or cone-like passage through which medicament may be delivered from the filled canister via the metering valve to the nose or mouth of a patient e.g. a mouthpiece actuator.

MDI canisters generally comprise a container capable of withstanding the vapour pressure of the propellant used such as a plastic or plastics-coated glass bottle or preferably a metal canister, for example, of aluminium or an alloy thereof which may optionally be anodised, lacquer-coated and/or plastic-coated (e.g. incorporated herein by reference WO96/32150 wherein part or all of the internal surfaces of the can are coated with one or more fluorocarbon polymers optionally in combination with one or more non-fluorocarbon polymers).

The cap may be secured onto the canister via welding such as ultrasonic welding or laser welding, screw fitting or crimping. MDIs taught herein may be prepared by methods of the art (e.g., see Byron, above and WO/96/32150). Preferably the canister is fitted with a cap assembly, wherein a formulation metering valve is situated in the cap, and said cap is crimped in place.

A further aspect of the invention is a sealed container as described above capable of withstanding the pressure required to maintain the propellant as a liquid, such as a metered dose inhaler, containing therein an aerosol formulation as described above.

The formulations of the invention may be prepared by dispersal of the medicament in the selected propellant in an appropriate container, for example, with the aid of sonication or a high-shear mixer. The process is desirably carried out under controlled humidity conditions.

The chemical and physical stability and the pharmaceutical acceptability of the aerosol formulations according to the invention may be determined by techniques well known to those skilled in the art. Thus the chemical stability of the components may be determined by HPLC assay, for example, after prolonged storage of the product. Physical stability data may be gained from other conventional analytical techniques such as by leak testing, by valve delivery assay (average shot weights per actuation), by dose reproducibility assay (active ingredient per actuation) and spray distribution analysis.

The suspension stability of the aerosol formulations according to the invention may be measured by conventional techniques, for example, by measuring flocculation size distribution using a back light scattering instrument or by measuring particle size distribution by cascade impaction or by the "twin impinger" analytical process. As used herein reference to the "twin impinger" assay means "Determination of the deposition of the emitted dose in pressurised inhalations using apparatus A" as defined in British Pharmacopaeia 1988, pages A204-207, Appendix XVII C. Such techniques enable the "respirable fraction" of the aerosol formulations to be calculated. One method used to calculate the "respirable fraction" is by reference to "fine particle fraction" which is the amount of active ingredient collected in the lower impingement chamber per actuation expressed as a percentage of the total amount of active ingredient delivered per actuation using the twin impinger method described above.

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto an aluminium can to form an empty canister. The particulate medicament is added to a charge vessel and liquefied propellant is pressure filled through the charge vessel into a manufacturing vessel, together with liquefied propellant containing the surfactant. The drug suspension is mixed before recirculation to a filling machine and an aliquot of the drug suspension is then filled through the metering valve into the canister.

In an alternative process, an aliquot of the liquefied formulation is added to an open canister under conditions which are sufficiently cold such that the formulation does not vaporise, and then a metering valve crimped onto the canister.

Typically, in batches prepared for pharmaceutical use, each filled canister is check-weighed, coded with a batch number and packed into a tray for storage before release testing.

Each filled canister is conveniently fitted into a suitable channelling device, prior to use, to form a metered dose inhaler system for administration of the medicament into the lungs or nasal cavity of a patient. Metered dose inhalers are designed to deliver a fixed unit dosage of medicament per actuation or "puff", for example, in the range of 10 to 5000 micrograms of medicament per puff.

Administration of medicament may be indicated for the treatment of mild, moderate, severe acute or chronic symptoms or for prophylactic treatment. It will be appreciated that the precise dose administered will depend on the age and condition of the patient, the particular particulate medicament used and the frequency of administration and will ultimately be at the discretion of the attendant physician. When combinations of medicaments are employed the dose of each component of the combination will in general be that employed for each component when used alone. Typically, administration may be one or more times, for example, from 1 to 8 times per day, giving for example 1, 2, 3 or 4 puffs each time.

Suitable daily doses, may be, for example in the range 50 to 200 micrograms of salmeterol, depending on the severity of the disease.

Thus, for example, each valve actuation may deliver 25 micrograms of salmeterol (as xinafoate). Typically each filled canister for use in a metered dose inhaler system contains 60, 100, 120, 160 or 240 metered doses or puffs of medicament.

An appropriate dosing regime for other medicaments will be known or readily available to persons skilled in the art.

A further aspect of the invention provides a method of reducing drug deposition, in a pharmaceutical aerosol formulation consisting essentially of particulate medicament, e.g salmeterol xinafoate optionally in combination with another drug useful in inhalation therapy, and a liquefied propellant which is 1,1,1,2,3,3,3-heptafluoro-n-propane, 1,1,1,2-tetrafluoroethane or mixtures thereof or mixtures thereof, on valve components, especially in a metering chamber and/or sealing gaskets for use in a MDI, comprising use of at least one sealing gasket composed of greater than 90% of a polymer of EPDM and presenting a substantially fluorinated metering chamber surface to the pharmaceutical hydrofluorocarbon aerosol formulation contained therein.

A further aspect of the invention is use of EPDM polymer in the preparation of a sealing gasket which when used in conjunction with a valve with a substantially fluorinated metering chamber surface and pharmaceutical aerosol formulation consists of or consisting essentially of particulate medicament, e.g. of salmeterol xinafoate, and a liquid propellant which is 1,1,1,2,3,3,3-heptafluoro-n-propane, 1,1,1,2-tetrafluoroethane or mixtures thereof provides the advantages described above.

The invention thus provides an MDI comprising a container, a described above, fitted with a suitable channelling device.

The use of an MDI as described above in inhalation therapy, for the treatment or prophylaxis of respiratory disorders is an alternative aspect of the invention. Specifically the MDI system, as described above, may be used in the treatment or prophylaxis of asthma or COPD.

Furthermore the invention includes a method of treating respiratory disorders such as asthma or COPD which comprises use of an MDI as described above by a patient.

Furthermore a package comprising an MDI as described above within a flexible wrapper, said wrapper composed of a material which is substantially permeable to evacuation of propellant gas and substantially impermeable to intrusion of atmospheric moisture e.g. as described in USP 6, 119, 853 is another aspect of the invention.

Preferably the package will also contain within it a desiccant material. The desiccant material may be inside the MDI system and/or outside the MDI system.

In a further aspect the invention provides a container suitable for containing a pharmaceutical aerosol formulation comprising a canister sealed with a metering valve, said valve comprising a metering chamber having an upper and a lower sealing gasket and a valve stem, wherein the valve is sealed to the canister by means of a neck sealing gasket, characterised in that at least one gasket is composed of greater than 90% of a polymer of EPDM and the metering chamber surface presents a substantially fluorinated surface to the formulation.

Especially preferred is a container as described above wherein the metering valve comprises a metering chamber (4) defined by walls and an upper (12) and a lower (9) sealing gasket through which pass a valve stem (7 and 8) characterised in that said two sealing gaskets are composed of greater than 90% of a polymer of EPDM and the metering chamber surface presents a substantially fluorinated surface to a formulation containable therein.

Also especially preferred is a container as described above wherein the valve is sealed to the canister by means of a can sealing gasket (3) which is composed of greater than 90% of EPDM polymer optionally wherein the lower (9) sealing gasket is also composed of greater than 90% of EPDM polymer.

Most preferably all the sealing gaskets in the said metering valve are composed of greater than 90% of EPDM polymer.

Furthermore usually the surface of surface of the metering chamber will be treated so as to present a substantially fluorinated surface to the formulation.

Thus the invention encompasses a container suitable for containing a pharmaceutical aerosol formulation comprising a canister sealed with a metering valve, said valve comprising a metering chamber having an upper and a lower sealing gasket and a valve stem characterised in that (i) the valve is sealed to the canister by means of a neck sealing gasket composed of greater than 90% of a polymer of EPDM; (ii) said upper and lower metering chamber sealing gaskets are composed of greater than 90% of a polymer of EPDM and (iii) the metering chamber is surface treated so as to present a substantially fluorinated surface to the formulation.

Additionally the invention provides a metering valve, and use thereof, suitable for dispensing a pharmaceutical aerosol formulation comprising a metering chamber having an upper and a lower sealing gasket and a valve stem, characterised in that at least one gasket is composed of greater than 90% of a polymer of EPDM and the metering chamber surface presents a substantially fluorinated surface to the formulation.

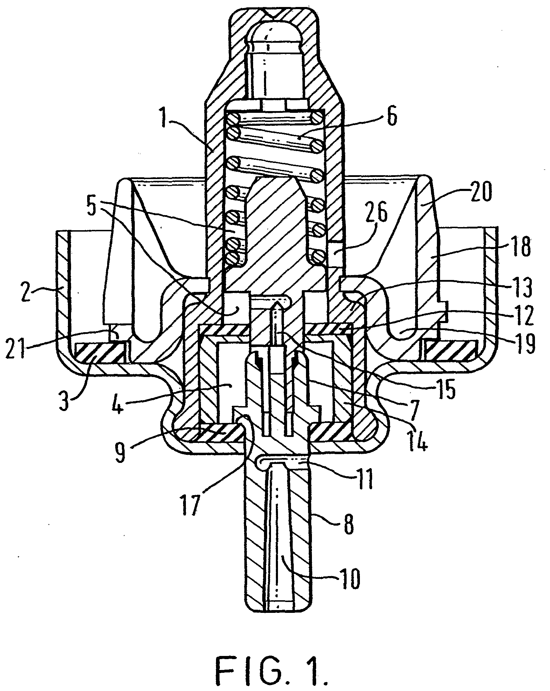

An exemplary valve of use according to the invention is shown in Figure 1 and comprises a valve body 1 sealed in a ferrule 2 by means of crimping, the ferrule itself being set on the neck of a container (not shown) with interposition of a gasket 3 (can seal) in a well-known manner.

The valve body 1 is formed at its lower part with a metering chamber 4, and its upper part with a sampling chamber 5 which also acts as a housing for a return spring 6. The metering chamber is constructed from a fluorinated polymer at least in part and/or a fluorinated coating according to the invention. The words "upper" and "lower" are used for the container when it is in a use orientation with the neck of the container and valve at the lower end of the container which corresponds to the orientation of the valve as shown in Figure 1. Inside the valve body 1 is disposed a valve stem 7, a part 8 of which extends outside the valve through lower stem seal 9 and ferrule 2. The stem part 8 is formed with an inner axial or longitudinal canal 10 opening at the outer end of the stem and in communication with a radial passage 11.

The upper portion of stem 7 has a diameter such that it can slide through an opening in an upper stem seal 12 and will engage the periphery of that opening sufficiently to provide a seal. Upper stem seal 12 is held in position against a step 13 formed in the valve body 1 between the said lower and upper parts by a sleeve 14 which defines the metering chamber 4 between lower stem seal 9 and upper stem seal 12. The valve stem 7 has a passage 15 which, when the stem is in the inoperative position shown, provides a communication between the metering chamber 4 and sampling chamber 5, which itself communicates with the interior of the container via orifice 26 formed in the side of the valve body 1.

Valve stem 7 is biased downwardly to the inoperative position by return spring 6 and is provided with a shoulder 17 which abuts against lower stem seal 9. In the inoperative position as shown in Figure 1 shoulder 17 abuts against lower stem seal 9 and radial passage 11 opens below lower stem seal 9 so that the metering chamber 4 is isolated from canal 10 and suspension inside cannot escape.

A ring 18 having a "U" shaped cross section extending in a radial direction is disposed around the valve body below orifice 26 so as to form a trough 19 around the valve body.

As seen in Figure 1 the ring is formed as a separate component having an inner annular contacting rim of a diameter suitable to provide a friction fit over the upper part of valve body 1, the ring seating against step 13 below the orifice 26. However, the ring 18 may alternatively be formed as an integrally moulded part of valve body 1.

To use the device the container is first shaken to homogenise the suspension within the container. The user then depresses the valve stem 7 against the force of the spring 6.

When the valve stem is depressed both ends of the passage 15 come to lie on the side of upper stem seal 12 remote from the metering chamber 4. Thus a dose is metered within the fluorinated metering chamber. Continued depression of the valve stem will move the radial passage 11 into the metering chamber 4 while the upper stem seal 12 seals against the valve stem body. Thus, the metered dose can exit through the radial passage 11 and the outlet canal 10.

Releasing the valve stem causes it to return to the illustrated position under the force of the spring 6. The passage 15 then once again provides communication between the metering chamber 4 and sampling chamber 6. Accordingly, at this stage liquid passes under pressure from the container through orifice 26, through the passage 15 and thence into the metering chamber 4 to fill it.

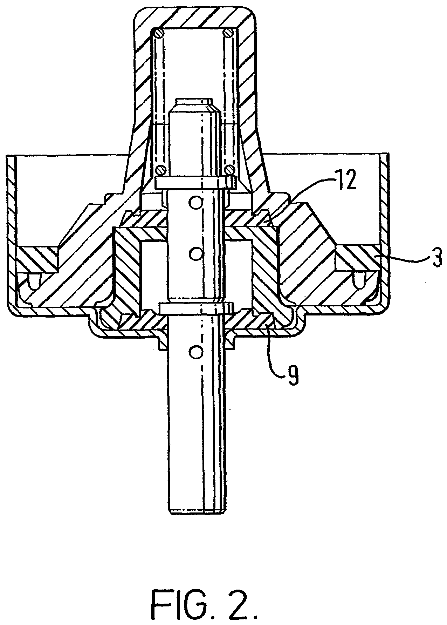

Figure 2 shows a different view of a valve in which the gasket seal and lower and upper stem seals are labelled 3, 9 and 12 respectively.

The invention will now be described further with reference the following Example which serve to illustrate the invention.

### Example

### Sample Preparation

The MDIs for which data are presented in Tables 1 to 5 were prepared in aluminium canisters coated with a PTFE/PES polymer blend as described in WO96/32150 and sealed with a Bespak valve wherein all the gaskets were made from conventional nitrile rubber (as comparator) or EPDM polymer (according to the invention) and wherein the metering chamber is composed of PBT and was conventional or surface treated with a plasma coating of a C₁₋₁₀perfluoroalkane.

Furthermore the said aluminium canisters contained a pharmaceutical aerosol formulation comprising 4.2mg of salmeterol xinafoate and 12g of HFA 134a.

Each device was stored at 40°C and 75% relative humidity unless otherwise stated.

### Method for Determining Total Drug Content (TDC) in MDIs containing Salmeterol Xinafoate and HFA 134a

Each MDI canister tested (before use) was cooled in a freezing mixture of dry ice and methanol for approximately 5 minutes, after which it was clamped and the valve assembly removed with a suitable tube cutter. The contents of the can was quantitatively transferred into a receptacle(s) of known volume and the can, valve and valve components quantitatively washed. The combined can contents and associated washings were then assayed by HPLC and the TDC calculated. TDC values which are lower than predicted imply absorption of drug into valve components.

Mean can content is the weight of formulation contained in the canister calculated by mass difference.

### Method for Determining Dose and FPM

Each MDI canister tested was put into a clean actuator and primed by firing 4 shots.

Then 10 shots were fired into an Andersen Cascade Impactor which was quantitatively washed and the amount of drug deposited thereon quantified by HPLC analysis of the washings.

From this the dose delivered (the sum of the amount of drug deposited on the cascade impactor) and the FPM (the sum of drug deposited on stages two 3, 4 and 5) data were calculated. Values of FPM which are lower than expected imply one or more of the following: (i) absorption, (ii) deposition or (iii) particle growth.

The mean dose delivered is the mean of 3 dose delivered determinations. The %FPM ex-device is a measure of the dose available to the patient.

On visual inspection it was observed that the drug substance obtained from the conventional MDIs stored at 40°C 20% RH (i.e. with nitrile gaskets and a normal metering chamber [as shown in Table 1]) had the same appearance and appeared unchanged from the initial timepoint. However the drug substance from conventional MDIs stores at 40°C 75% RH was distinctly crystalline in appearance indicating some dissolution and recrystallisation.

Table 1 shows that TDC values obtained for MDIs obtained for conventional MDIs stored at stored at 40°C 75% RH and 40°C 20% RH. The former had a significantly lower TDC valve than the initial timepoint and those stored under low humidity conditions.

Table 2 shows the dose delivered by the conventional MDI (control) is reduced on storage at 40°C 75% RH. The trend is very evident by the 6/7 month timepoint. The trend is not observed in MDIs wherein all the gaskets are prepared from EPDM polymer. The trend may be present in the MDIs with a plasma treated metering chamber, however if present the trend seems to be not so pronounced as for the conventional MDI.

The FPM data for the conventional MDI employing nitrile gaskets shows a significant decrease after storage at 40°C 75% RH. This trend is reduced noticeably in addition to the initial timepoint value being higher in the MDI where all the gaskets are prepare from EPDM polymer. The data for the MDI with a plasma treated metering chamber seems to indicate an initial value for FPM higher than both the control and the EPDM polymer MDIs. However the data suggests that this value is reduced on storage at 40°C 75% RH albeit by not as much as the reduction observed for the conventional MDI.

The data in Table 3 supports the trends observed in Table 2.

The data in Table 4 shows that MDIs with gaskets constructed from a polymer of EPDM and having a metering chamber with a fluorinated surface provide an increase, in µg, in the dose delivered and practically eliminate the fall in dose delivered, observed on storage of the product especially under high humidity conditions, whilst simultaneously minimising the reduction in FPM observed, in comparison to conventional MDIs or those with either gaskets constructed from a polymer of EPDM or having a metering chamber with a fluorinated surface.

The data in Table 5 supports the trends observed in Table 4.

The throat piece used in the Andersen Cascade Impactor to generate data contained in Table 4 and Table 5 was of the USP type. Therefore although the data was obtained using the same procedure as described above for Table 2 and Table 3 it is not directly comparable to the latter, wherein a throat manufactured for Glaxo Wellcome was used.

From the Tables it can be concluded that use of EPDM gaskets and metering chambers with a fluorinated surface in MDIs containing a pharmaceutical aerosol formulation of particulate medicament, especially salmeterol xinafoate in a liquefied HFA propellant results in a formulation with improved stability in comparison to either conventional MDIs or those containing EDPM gaskets or metering chambers plasma coated with a fluorinated coating.

**TABLE 1 EFFECT OF STORAGE CONDITION ON TOTAL DRUG CONTENT OF SALMETEROL INHALER**

| **Metering Chamber** | **RUBBER TYPE** | **Storage Time Months** | **Storage Condition** | **Mean TDC (mg)** | **Mean Can Content (g)** |
|---|---|---|---|---|---|
| Normal | Nitrile | 10 | 40°C/75%RH | 3.6 | 11.5 |
| Normal | Nitrile | 10 | 40°/20%RH | 4.1 | 11.5 |

NOTES: All results are the mean of three individual results and TDC at initial timepoint around 4.2mg

**TABLE 2 EFFECT OF STORAGE CONDITION ON DOSE DELIVERED AND FPM OF SALMETEROL INHALER**

| | | **Dose Delivered (µg)** | | | **FPM (µg)** | | |
|---|---|---|---|---|---|---|---|
| **Metering Chamber** | **Rubber Type** | **Initial** | **6 weeks** | **6/7 Months** | **Initial** | **6 weeks** | **617 Months** |
| Normal | Nitrile | 18.5 | 16.8 | 13.4 | 9.3 | 7.2 | 5.0 |
| Normal | EPDM | 19.8 | 18.7 | 20.1 | 11.2 | 10.7 | 10.5 |
| Plasma Treated | Nitrile | 21.6 | - | 19.7 | 13.0 | - | 10.3 |

**TABLE 3**

| **EFFECT OF STORAGE CONDITION ON MEAN DOSE DELIVERED & RANGE OF DOSE DELIVERED FOR SALMETEROL INHALERS** | | | | | | | |
|---|---|---|---|---|---|---|---|
| | | **Mean Dose Delivered (µg)** | | | **Range of Dose Delivered (µg)** | | |
| **Metering Chamber** | **Rubber Type** | **Initial** | **6 weeks** | **6/7 Months** | **Initial** | **6 weeks** | **6/7 Months** |
| Normal | Nitrile | 19.1 | 16.8 | 14.5 | 17.1-20.7 | 15.4-19.2 | 12.8-16.1 |
| Normal | EPDM | 19.0 | 19.1 | 18.9 | 17.0-19.7 | 17.8-20.1 | 18.1-19.6 |
| Plasma Treated | Nitrile | 21.2 | 21.0 | 18.4 | 19.9-22.1 | 20.5-21.5 | 18.0-19.4 |

**TABLE 4 EFFECT OF STORAGE CONDITION ON DOSE DELIVERED AND FPM OF SALMETEROL INHALER**

| | | **Dose Delivered (µg)** | | | **FPM (µg)** | | | **FPM as a % of ex device Dose** | | |
|---|---|---|---|---|---|---|---|---|---|---|
| **Metering Chamber** | **Rubber Type** | **Initial** | **Months** | | **Initial** | **Months** | | **Initial** | **Months** | |
| | | | **3** | **6** | | **3** | **6** | | **3** | **6** |
| Normal | Nitrile | 17.5 | 15.3 | 14.7 | 8.3 | 6.3 | 5.8 | 47 | 41 | 39 |
| Normal | EPDM | 18.5 | 17.9 | 17.7 | 9.3 | 8.4 | 8.0 | 50 | 47 | 45 |
| Plasma Treated | Nitrile | 19.9 | 19.0 | 16.8 | 11.1 | 8.4 | 6.5 | 56 | 44 | 39 |
| Plasma Treated | EPDM | 20.3 | 20.9 | 20.2 | 10.9 | 11.0 | 9.9 | 54 | 53 | 49 |

**TABLE 5**

| **EFFECT OF STORAGE CONDITION ON MEAN DOSE DELIVERED & RANGE OF DOSE DELIVERED FOR SALMETEROL INHALERS** | | | | | | | |
|---|---|---|---|---|---|---|---|
| | | **Mean Dose Delivered (µg)** | | | **Range of Dose Delivered (µg)** | | |
| **Metering Chamber** | **Rubber Type** | **Initial** | **3 Months** | **6 Months** | **Initial** | **3 Months** | **6 Months** |
| Normal | Nitrile | 18.1 | 16.0 | 14.6 | 17.3-19.2 | 14.9-18.3 | 13.4-16.4 |
| Normal | EPDM | 18.3 | 18.7 | 17.4 | 16.8-19.9 | 17.7-19.5 | 16.4-19.2 |
| Plasma Treated | Nitrile | 19.7 | 19.1 | 17.2 | 19.2-20.7 | 18.2-19.7 | 16.4-18.1 |
| Plasma Treated | EPDM | 20.3 | 20.3 | 20.4 | 19.4-21.2 | 19.9-21.1 | 19.4-21.0 |

## Claims

1. A container comprising a canister sealed with a metering valve, having a metering chamber, which contains a pharmaceutical aerosol formulation consisting essentially of
(A) particulate salmeterol xinafoate optionally in combination with another drug useful in inhalation therapy, suspended in
(B) a liquefied propellant gas comprising 1,1,1,2,3,3,3-heptafluoro-n-propane, 1,1,1,2-tetrafluoroethane or a mixture thereof;
wherein the formulation contains less than 0.01% of surfactant based on weight of medicament and contains less than 0.01% of components having polarity higher than the liquefied propellant gas based on weight of medicament;
said valve **characterised in that** it contains one or more sealing gaskets composed of greater than 90% of a polymer of ethylene propylene diene monomer (EPDM) and the metering chamber surface presents a substantially fluorinated surface to the formulation.

2. A container as claimed in claim 1 wherein the liquefied propellant gas is 1,1,1,2-tetrafluoroethane.

3. A container as claimed in claim 1 or claim 2 wherein the another drug useful in inhalation therapy is fluticasone propionate or ipratropium bromide.

4. A container as claimed in claim 1 or claim 2 wherein salmeterol xinafoate is the only medicament.

5. A container according to any one of claims 1 to 4 wherein the valve is sealed to the canister by means of a neck sealing gasket which is composed of greater than 90% of a polymer of ethylene propylene diene monomer (EPDM).

6. A container according to any one of claims 1 to 5 wherein the metering valve includes a metering chamber having an upper and a lower sealing gasket and a valve stem **characterised in that** said two sealing gaskets are composed of greater than 90% of a polymer of ethylene propylene diene monomer (EPDM).

7. A container according to any one of claims 1 to 6 wherein the metering chamber is constructed from a plastics material.

8. A container according to claim 7 wherein the plastics material is nylon, PBT or acetal.

9. A container according to any one of claims 1 to 8 wherein the metering chamber is surface treated so as to present a substantially fluorinated surface to the formulation.

10. A container according to claim 9 wherein the surface treatment comprises a process of plasma coating with a C₁₋₁₀perfluoroalkane.

11. A container according to claim 7 wherein the metering chamber is constructed from a material selected from the group consisiting of a polyethylenetetrafluoroethylene, a polyvinyldienefluoride, a polyperfluoroalkoxyalkane, a polychlorotrifluoroethylene, a fluorinated polyethylene propylene, a copolymer of a polytetrafluoroethylene and a polyperfluoroalkoxyalkane, a copolymer of a polytetrafluoroethylene and a polyhexafluoropropylene, a copolymer of a polyvinyldienefluoride and a polyhexafluoropropylene, a copolymer of a polytetrafluoroethylene and a polyperfluoro(propyl vinyl ether); a blend of a polytetrafluoroethylene, a polyhexafluoropropylene a polyvinylidene fluoride, blends thereof and combinations thereof.

12. A container according to any one of claims 1 to 6 wherein the metering chamber is composed of a metallic material.

13. A container according to claim 12 wherein the metallic material is aluminium or stainless steel.

14. A container according to claim 12 or 13 wherein the metering chamber is surface treated so as to present a substantially fluorinated surface to the formulation.

15. A container according to claim 14 wherein the surface treatment comprises a process of applying a coating of a fluorocarbon polymer optionally in combination with a non-fluorocarbon polymer.

16. A container according to claim 14 or claim 15 wherein fluorocarbon polymer is selected from FEP and PTFE.

17. A container according to any one of claims 14 to 16 wherein the coating is a coating of FEP.

18. A container according to any one of claims 14 to 16 wherein the coating is a coating of a blend of PTFE and PES.

19. A container according to any one of claims 1 to 18 wherein the canister is composed of aluminium.

20. A container according to claim 19 wherein the canister is surface treated so as to present a substantially fluorinated surface to the formulation.

21. A container according to claim 20 wherein the canister is surface treated by coating with a fluorocarbon polymer optionally in combination with a non-fluorocarbon polymer.

22. A container according to claim 20 or claim 21 wherein fluorocarbon polymer is selected from FEP and PTFE.

23. A container according to any one of claims 20 to 22 wherein the coating is a coating of FEP.

24. A container according to any one of claims 20 to 22 wherein the coating is a coating of a blend of PTFE and PES.

25. A metered dose inhaler comprising a container according to any one of claims 1 to 24 fitted with a suitable channelling device.

26. Use of a metered dose inhaler according to claim 25 in the manufacture of a medicament for the treatment of asthma or COPD.

27. A package comprising a metered dose inhaler according to claim 25 contained within a flexible wrapper said wrapper composed of a material which is substantially permeable to evacuation of propellant gas and impermeable to intrusion of atmospheric moisture.

28. A package according to claim 27 **characterised in** the flexible wrapper also contains within it a desiccant material.

29. A package according to claim 28 **characterised in** the can contains within it a desiccant material.

30. A container suitable for containing a pharmaceutical aerosol formulation comprising a canister sealed with a metering valve, said valve comprising a metering chamber having an upper and a lower sealing gasket and a valve stem, wherein the valve is sealed to the canister by means of a neck sealing gasket, **characterised in that** at least one gasket is composed of greater than 90% of a polymer of ethylene propylene diene monomer (EPDM) and the metering chamber surface presents a substantially fluorinated surface to the formulation.

31. A container suitable for containing a pharmaceutical aerosol formulation according to claim 30 wherein the upper, lower and neck sealing gaskets are composed of greater than 90% of a polymer of ethylene propylene diene monomer (EPDM).

32. A container suitable for containing a pharmaceutical aerosol formulation according to claim 30 or claim 31 wherein the metering chamber is surface treated so as to present a substantially fluorinated surface to the formulation.

33. A container according to claim 32 wherein the surface treatment comprises a process of plasma coating with a C₁₋₁₀perfluoroalkane.

34. A container according to any one of claims 30 to 33 wherein the metering chamber is constructed from a plastics material.

35. A container according to claim 34 wherein the plastics material is nylon, PBT or acetal.

36. A container according to any one of claims 30 to 35 wherein the canister is composed of aluminium.

37. A container according to claim 36 wherein the canister is surface treated so as to present a substantially fluorinated surface to the formulation.

38. A container according to claim 37 wherein the canister is surface treated by coating with a fluorocarbon polymer optionally in combination with a non-fluorocarbon polymer.

39. A container according to any one of claims 30 to 38 which contains a pharmaceutical aerosol formulation comprising a particulate medicament and a liquefied propellant gas of 1,1,1,2,3,3,3-heptafluoro-n-propane, 1,1,1,2-tetrafluoroethane or mixtures thereof.

40. A container according to claim 39 wherein the propellant gas is 1,1,1,2-tetrafluoroethane.

41. A container according to claim 39 or claim 40 wherein the particulate medicament is selected from salmeterol xinafoate, fluticasone propionate, albuterol or a salt thereof, beclomethasone dipropionate, formoterol or a salt thereof, ipratropium bromide, budesonide, sodium cromoglycate and combinations thereof.

42. A container according to claim 41 wherein the particulate medicament is salmeterol xinafoate optionally in combination with fluticasone propionate.

## Patentansprüche

1. Behälter, der einen mit einem Dosierventil verschlossenen Kanister umfasst und der eine Dosierkammer hat, die eine pharmazeutische Aerosolformulierung enthält, die im Wesentlichen aus
(A) teilchenförmigem Salmeterolxinafoat gegebenenfalls in Kombination mit einem anderen in der Inhalationstherapie verwendbaren Arzneimittel, suspendiert in
(B) einem verflüssigten Treibgas, das 1,1,1,2,3,3,3-Heptafluor-n-propan, 1,1,1,2-Tetrafluorethan oder eine Mischung davon umfasst;
wobei die Formulierung weniger als 0.01% an Tensid bezogen auf das Gewicht des Medikaments enthält und weniger als 0.01% an Komponenten mit einer Polarität höher als das verflüssigte Treibgas, bezogen auf das Gewicht des Medikaments, enthält; das Ventil **dadurch gekennzeichnet ist, dass** es eine oder mehrere Dichtungen enthält, die aus mehr als 90% eines Polymers aus Ethylen-Propylen-Dien Monomer (EPDM) zusammengesetzt sind, und die Dosierkammeroberfläche eine im Wesentlichen fluorierte Oberfläche für die Formulierung darstellt;
besteht.

2. Behälter nach Anspruch 1, wobei das verflüssigte Treibgas 1,1,1,2-Tetrafluorethan ist.

3. Behälter nach Anspruch 1 oder Anspruch 2, wobei das andere in der Inhalationstherapie verwendbare Arzneimittel Fluticasonpropionat oder Ipratropiumbromid ist.

4. Behälter nach Anspruch 1 oder Anspruch 2, wobei Salmeterolxinafoat das einzige Medikament ist.

5. Behälter gemäß mindestens einem der Ansprüche 1 bis 4, wobei das Ventil mit dem Kanister mittels einer Hals-Dichtung, die aus mehr als 90% eines Polymers aus Ethylen-Propylen-Dien Monomer (EPDM) zusammengesetzt ist, verschlossen ist.

6. Behälter gemäß mindestens einem der Ansprüche 1 bis 5, wobei das Dosierventil eine Dosierkammer mit einer oberen und einer unteren Dichtung und einen Ventilschaft enthält, **dadurch gekennzeichnet, dass** die zwei Dichtungen aus mehr als 90% eines Polymers aus Ethylen-Propylen-Dien Monomer (EPDM) zusammengesetzt sind.

7. Behälter gemäß mindestens einem der Ansprüche 1 bis 6, wobei die Dosierkammer aus einem Kunststoffmaterial aufgebaut ist.

8. Behälter gemäß Anspruch 7, wobei das Kunststoffmaterial Nylon, PBT oder Acetal ist.

9. Behälter gemäß mindestens einem der Ansprüche 1 bis 8, wobei die Dosierkammer oberflächenbehandelt ist, sodass sie eine im Wesentlichen fluorierte Oberfläche für die Formulierung darstellt.

10. Behälter gemäß Anspruch 9, wobei die Oberflächenbehandlung ein Verfahren des Plasma-Beschichtens mit einem C₁₋₁₀-Perfluoralkan umfasst.

11. Behälter gemäß Anspruch 7, wobei die Dosierkammer aus einem Material aufgebaut ist, ausgewählt aus der Gruppe bestehend aus einem Polyethylentetrafluorethylen, einem Polyvinyldienfluorid, einem Polyperfluoralkoxyalkan, einem Polychlortrifluorethylen, einem fluorierten Polyethylenpropylen, einem Copolymer eines Polytetrafluorethylens und eines Polyperfluoralkoxyalkans, einem Copolymer eines Polytetrafluorethylens und eines Polyhexafluorpropylens, einem Copolymer eines Polyvinyldienfluorids und eines Polyhexafluorpropylens, einem Copolymer eines Polytetrafluorethylens und eines Polyperfluor(propylvinylethers); einer Mischung eines Polytetrafluorethylens, eines Polyhexafluorpropylens und eines Polyvinylidenfluorids, Mischungen davon und Kombinationen davon.

12. Behälter gemäß mindestens einem der Ansprüche 1 bis 6, wobei die Dosierkammer aus einem metallischen Material zusammengesetzt ist.

13. Behälter gemäß Anspruch 12, wobei das metallische Material Aluminium oder rostfreier Stahl ist.

14. Behälter gemäß Anspruch 12 oder 13, wobei die Dosierkammer oberflächenbehandelt ist, um eine im Wesentlichen fluorierte Oberfläche für die Formulierung darzustellen.

15. Behälter gemäß Anspruch 14, wobei die Oberflächenbehandlung ein Verfahren zum Aufbringen einer Beschichtung eines Fluorkohlenstoffpolymers, gegebenenfalls in Kombination mit einem Nicht-Fluorkohlenstoffpolymer, umfasst.

16. Behälter gemäß Anspruch 14 oder Anspruch 15, wobei das Fluorkohlenstoffpolymer ausgewählt ist aus FEP und PTFE.

17. Behälter gemäß mindestens einem der Ansprüche 14 bis 16, wobei die Beschichtung eine Beschichtung aus FEP ist.

18. Behälter gemäß mindestens einem der Ansprüche 14 bis 16, wobei die Beschichtung eine Beschichtung aus einer Mischung aus PTFE und PES ist.

19. Behälter gemäß mindestens einem der Ansprüche 1 bis 18, wobei der Kanister aus Aluminium zusammengesetzt ist.

20. Behälter gemäß Anspruch 19, wobei der Kanister oberflächenbehandelt ist, um eine im Wesentlichen fluorierte Oberfläche für die Formulierung darzustellen.

21. Behälter gemäß Anspruch 20, wobei der Kanister durch Beschichten mit einem Fluorkohlenstoffpolymer, gegebenenfalls in Kombination mit einem Nicht-Fluorkohlenstoffpolymer, oberflächenbehandelt ist.

22. Behälter gemäß Anspruch 20 oder Anspruch 21, wobei das Fluorkohlenstoffpolymer ausgewählt ist aus FEP und PTFE.

23. Behälter gemäß mindestens einem der Ansprüche 20 bis 22, wobei die Beschichtung eine Beschichtung aus FEP ist.

24. Behälter gemäß mindestens einem der Ansprüche 20 bis 22, wobei die Beschichtung eine Beschichtung aus einer Mischung aus PTFE und PES ist.

25. Dosierinhalator, der einen Behälter gemäß mindestens einem der Ansprüche 1 bis 24, versehen mit einer geeigneten Kanalisierungsvorrichtung, umfasst.

26. Verwendung eines Dosierinhalators gemäß Anspruch 25 bei der Herstellung eines Medikaments zur Behandlung von Asthma oder COPD.

27. Verpackung, die einen Dosierinhalator gemäß Anspruch 25 umfasst, der in einer flexiblen Umhüllung enthalten ist, wobei die Umhüllung aus einem Material zusammengesetzt ist, das im Wesentlichen durchlässig für die Evakuierung von Treibgas ist und undurchlässig für das Eindringen von atmosphärischer Feuchtigkeit ist.

28. Verpackung gemäß Anspruch 27, **dadurch gekennzeichnet, dass** in der flexiblen Umhüllung auch ein Trockenmittel enthalten ist.

29. Verpackung gemäß Anspruch 28, **dadurch gekennzeichnet, dass** in der Dose ein Trockenmittel enthalten ist.

30. Behälter geeignet zur Aufnahme einer pharmazeutischen Aerosolformulierung, der einen Kanister mit einem Dosierventil umfasst, wobei das Ventil eine Dosierkammer, die eine obere und eine untere Dichtung und einen Ventilschaft enthält, umfasst, wobei das Ventil mit dem Kanister mittels einer Hals-Dichtung verschlossen ist, die **dadurch gekennzeichnet ist, dass** wenigstens eine Dichtung aus mehr als 90% eines Polymers aus Ethylen-Propylen-Dien Monomer (EPDM) zusammengesetzt ist und die Dosierkammeroberfläche eine im Wesentlichen fluorierte Oberfläche für die Formulierung darstellt.

31. Behälter geeignet zur Aufnahme einer pharmazeutischen Aerosolformulierung gemäß Anspruch 30, wobei die obere, untere und Hals-Dichtungen aus mehr als 90% eines Polymers aus Ethylen-Propylen-Dien Monomer (EPDM) zusammengesetzt sind.

32. Behälter geeignet zur Aufnahme einer pharmazeutischen Aerosolformulierung gemäß Anspruch 30 oder Anspruch 31, wobei die Dosierkammer oberflächenbehandelt ist, um eine im Wesentlichen fluorierte Oberfläche für die Formulierung darzustellen.

33. Behälter gemäß Anspruch 32, wobei die Oberflächenbehandlung ein Verfahren des Plasma-Beschichtens mit einem C₁₋₁₀-Perfluoralkan umfasst.

34. Behälter gemäß mindestens einem der Ansprüche 30 bis 33, wobei die Dosierkammer aus einem Kunststoffmaterial aufgebaut ist.

35. Behälter gemäß Anspruch 34, wobei das Kunststoffmaterial Nylon, PBT oder Acetal ist.

36. Behälter gemäß mindestens einem der Ansprüche 30 bis 35, wobei der Behälter aus Aluminium zusammengesetzt ist.

37. Behälter gemäß Anspruch 36, wobei der Kanister oberflächenbehandelt ist, um eine im Wesentlichen fluorierte Oberfläche für die Formulierung darzustellen.

38. Behälter gemäß Anspruch 37, wobei der Kanister durch Beschichten mit einem Fluorkohlenstoffpolymer, gegebenenfalls in Kombination mit einem Nicht-Fluorkohlenstoffpolymer, oberflächenbehandelt ist.

39. Behälter gemäß mindestens einem der Ansprüche 30 bis 38, der eine pharmazeutische Aerosolformulierung enthält, die ein teilchenförmiges Medikament und ein verflüssigtes Treibgas aus 1,1,1,2,3,3,3-Heptafluor-n-propan, 1,1,1,2-Tetrafluorethan oder Mischungen davon umfasst.

40. Behälter gemäß Anspruch 39, wobei das Treibgas 1,1,1,2-Tetrafluorethan ist.

41. Behälter gemäß Anspruch 39 oder Anspruch 40, wobei das teilchenförmige Medikament ausgewählt ist aus Salmeterolxinafoat, Fluticasonpropionat, Albuterol oder einem Salz davon, Beclomethasondipropionat, Formoterol oder einem Salz davon, Ipratropiumbromid, Budesonid, Natriumcromoglycat und Kombinationen davon.

42. Behälter gemäß Anspruch 41, wobei das teilchenförmige Medikament Salmeterolxinafoat, gegebenenfalls in Kombination mit Fluticasonpropionat, ist.

## Revendications

1. Contenant comprenant un réservoir scellé avec une valve doseuse, ayant une chambre de dosage, qui contient une formulation d'aérosol pharmaceutique constituée essentiellement
(A) de xinafoate de salmétérol particulaire facultativement en combinaison avec un autre remède utile en thérapie d'inhalation, mis en suspension dans
(B) un gaz propulseur liquéfié comprenant du 1,1,1,2,3,3,3-heptafluoro-n-propane, 1,1,1,2-tétrafluoroéthane ou un mélange de ceux-ci ;
dans lequel la formulation contient moins de 0,01 % de tensioactif par rapport au poids de médicament et contient moins de 0,01 % de composants ayant une polarité supérieure au gaz propulseur liquéfié par rapport au poids de médicament ;
ladite valve étant **caractérisée en ce qu'**elle contient un ou plusieurs joints de scellement composés de plus de 90 % d'un polymère de monomère éthylène-propylène-diène (EPDM) et la surface de chambre de dosage présente une surface sensiblement fluorée à la formulation.

2. Contenant selon la revendication 1, dans lequel le gaz propulseur liquéfié est le 1,1,1,2-tétrafluoroéthane.

3. Contenant selon la revendication 1 ou la revendication 2, dans lequel l'autre remède utile en thérapie d'inhalation est le propionate de fluticasone ou de bromure d'ipratropium.

4. Contenant selon la revendication 1 ou la revendication 2, dans lequel le xinafoate de salmétérol est le seul médicament.

5. Contenant selon l'une quelconque des revendications 1 à 4, dans lequel la valve est scellée au réservoir au moyen d'un joint de scellement de col qui est composé de plus de 90 % d'un polymère de monomère éthylène-propylène-diène (EPDM).

6. Contenant selon l'une quelconque des revendications 1 à 5, dans lequel la valve doseuse comporte une chambre de dosage ayant un joint de scellement supérieur et inférieur et une tige de valve, **caractérisé en ce que** lesdits deux joints de scellement sont composés de plus de 90 % d'un polymère de monomère éthylène-propylène-diène (EPDM).

7. Contenant selon l'une quelconque des revendications 1 à 6, dans lequel la chambre de dosage est construite à partir d'un matériau plastique.

8. Contenant selon la revendication 7, dans lequel le matériau plastique est le nylon, le PBT ou l'acétal.

9. Contenant selon l'une quelconque des revendications 1 à 8, dans lequel la chambre de dosage est traitée en surface de façon à présenter une surface sensiblement fluorée à la formulation.

10. Contenant selon la revendication 9, dans lequel le traitement de surface comprend un processus de revêtement au plasma avec un perfluoroalcane en C_{1 à 10}.

11. Contenant selon la revendication 7, dans lequel la chambre de dosage est construite à partir d'un matériau choisi dans le groupe constitué d'un polyéthylènetétrafluoroéthylène, un polyfluorure de vinyl diène, un polyperfluoroalcoxyalcane, un polychlorotrifluoroéthylène, un polyéthylène propylène fluoré, un copolymère d'un polytétrafluoroéthylène et d'un polyperfluoroalcoxyalcane, un copolymère d'un polytétrafluoroéthylène et d'un polyhexafluoropropylène, un copolymère d'un polyfluorure de vinyl diène et d'un polyhexafluoropropylène, un copolymère d'un polytétrafluoroéthylène et d'un polyperfluoro(propyl vinyl éther) ; un mélange d'un polytétrafluoroéthylène, d'un polyhexafluoropropylène et d'un polyfluorure de vinylidène, des mélanges de ceux-ci et des combinaisons de ceux-ci.

12. Contenant selon l'une quelconque des revendications 1 à 6, dans lequel la chambre de dosage est composée d'un matériau métallique.

13. Contenant selon la revendication 12, dans lequel le matériau métallique est l'aluminium ou l'acier inoxydable.

14. Contenant selon la revendication 12 ou 13, dans lequel la chambre de dosage est traitée en surface de façon à présenter une surface sensiblement fluorée à la formulation.

15. Contenant selon la revendication 14, dans lequel le traitement de surface comprend un processus d'application d'un revêtement d'un polymère fluorocarboné facultativement en combinaison avec un polymère non fluorocarboné.

16. Contenant selon la revendication 14 ou la revendication 15, dans lequel le polymère fluorocarboné est choisi parmi le FEP et le PTFE.

17. Contenant selon l'une quelconque des revendications 14 à 16, dans lequel le revêtement est un revêtement de FEP.

18. Contenant selon l'une quelconque des revendications 14 à 16, dans lequel le revêtement est un revêtement d'un mélange de PTFE et de PES.

19. Contenant selon l'une quelconque des revendications 1 à 18, dans lequel le réservoir est composé d'aluminium.

20. Contenant selon la revendication 19, dans lequel le réservoir est traité en surface de façon à présenter une surface sensiblement fluorée à la formulation.

21. Contenant selon la revendication 20, dans lequel le réservoir est traité en surface par revêtement avec un polymère fluorocarboné facultativement en combinaison avec un polymère non fluorocarboné.

22. Contenant selon la revendication 20 ou la revendication 21, dans lequel le polymère fluorocarboné est choisi parmi le FEP et le PTFE.

23. Contenant selon l'une quelconque des revendications 20 à 22, dans lequel le revêtement est un revêtement de FEP.

24. Contenant selon l'une quelconque des revendications 20 à 22, dans lequel le revêtement est un revêtement d'un mélange de PTFE et de PES.

25. Aérosol-doseur comprenant un contenant selon l'une quelconque des revendications 1 à 24 équipé d'un dispositif de canalisation approprié.

26. Utilisation d'un aérosol-doseur selon la revendication 25 dans la fabrication d'un médicament pour le traitement de l'asthme et de la broncho-pneumopathie chronique obstructive BPCO.

27. Conditionnement comprenant un aérosol-doseur selon la revendication 25 contenu dans un emballage flexible, ledit emballage étant composé d'un matériau qui est sensiblement perméable à l'évacuation de gaz propulseur et imperméable à l'intrusion d'humidité atmosphérique.

28. Conditionnement selon la revendication 27, **caractérisé en ce que** l'emballage flexible contient également un matériau déshydrateur à l'intérieur.

29. Conditionnement selon la revendication 28, **caractérisé en ce que** la boîte contient un matériau déshydrateur à l'intérieur.

30. Contenant approprié pour contenir une formulation d'aérosol pharmaceutique comprenant un réservoir scellé avec une valve doseuse, ladite valve comprenant une chambre de dosage ayant un joint de scellement supérieur et inférieur et une tige de valve, dans lequel la valve est scellée au réservoir au moyen d'un joint de scellement de col, **caractérisé en ce qu'**au moins un joint est composé de plus de 90 % d'un polymère de monomère éthylène-propylène-diène (EPDM) et la surface de la chambre de dosage présente une surface sensiblement fluorée à la formulation.

31. Contenant approprié pour contenir une formulation d'aérosol pharmaceutique selon la revendication 30, dans lequel les joints de scellement supérieur, inférieur et de col sont composés de plus de 90 % d'un polymère de monomère éthylène-propylène-diène (EPDM).

32. Contenant approprié pour contenir une formulation d'aérosol pharmaceutique selon la revendication 30 ou la revendication 31, dans lequel la chambre de dosage est traitée en surface de façon à présenter une surface sensiblement fluorée à la formulation.

33. Contenant selon la revendication 32, dans lequel le traitement de surface comprend un processus de revêtement au plasma avec un perfluoroalcane en C_{1 à 10}.

34. Contenant selon l'une quelconque des revendications 30 à 33, dans lequel la chambre de dosage est construite à partir d'un matériau plastique.

35. Contenant selon la revendication 34, dans lequel le matériau plastique est le nylon, le PBT ou l'acétal.

36. Contenant selon l'une quelconque des revendications 30 à 35, dans lequel le réservoir est composé d'aluminium.

37. Contenant selon la revendication 36, dans lequel le réservoir est traité en surface de façon à présenter une surface sensiblement fluorée à la formulation.

38. Contenant selon la revendication 37, dans lequel le réservoir est traité en surface par revêtement avec un polymère fluorocarboné facultativement en combinaison avec un polymère non fluorocarboné.

39. Contenant selon l'une quelconque des revendications 30 à 38, qui contient une formulation d'aérosol pharmaceutique comprenant un médicament particulaire et un gaz propulseur liquéfié de 1,1,1,2,3,3,3-heptafluoro-n-propane, de 1,1,1,2-tétrafluoroéthane ou de mélanges de ceux-ci.

40. Contenant selon la revendication 39, dans lequel le gaz propulseur est le 1,1,1,2-tétrafluoroéthane.

41. Contenant selon la revendication 39 ou la revendication 40, dans lequel le médicament particulaire est choisi parmi le xinafoate de salmétérol, le propionate de fluticasone, l'albutérol ou un sel de celui-ci, le dipropionate de beclométasone, le formotérol ou un sel de celui-ci, le bromure d'ipratropium, le budésonide, le cromoglycate de sodium et des combinaisons de ceux-ci.

42. Contenant selon la revendication 41, dans lequel le médicament particulaire est le xinafoate de salmétérol facultativement en combinaison avec le propionate de fluticasone.
